(19) Europäisches Patentamt
European Patent Office
Office européen des brevets

(11) Publication number: 0 084 292
B1

(12) EUROPEAN PATENT SPECIFICATION

(45) Date of publication of patent specification: 13.02.85

(51) Int. Cl.[4]: C 07 C 103/68, C 07 D 295/12, A 61 K 31/16, A 61 K 31/445, A 61 K 31/535

(21) Application number: 82810533.8

(22) Date of filing: 09.12.82

# (54) N,N-disubstituted alkenamides and phenylalkenamides, processes for their production and their use as pharmaceuticals.

(30) Priority: 14.12.81 US 330601

(43) Date of publication of application: 27.07.83 Bulletin 83/30

(45) Publication of the grant of the patent: 13.02.85 Bulletin 85/07

(84) Designated Contracting States: AT BE CH DE FR GB IT LI LU NL SE

(56) References cited:
EP-A-0 038 298

Chemical Abstracts, vol. 79, no. 25, 24 Dec 1973, Columbus, Ohio (USA); M.FLAMMANG et al.: "Esters and amides of substituted beta-phenoxycinnamic acids. Synthesis and pharmacological studies", p. 293, column 1, abstract 146118x

Chemical Abstracts, vol. 83, no. 23, 8 Dec 1975, Columbus, Ohio (USA); S.TANAKA et al.: "Cinnamic acid amides and their salts", p. 418, column 1, abstract 192858

(73) Proprietor: SANDOZ AG
Lichtstrasse 35
CH-4002 Basel (CH)
(84) BE CH FR GB IT LI LU NL SE

(73) Proprietor: SANDOZ-PATENT-GMBH
Humboldtstrasse 3
D-7850 Lörrach (DE)
(84) DE

(73) Proprietor: SANDOZ-ERFINDUNGEN Verwaltungsgesellschaft m.b.H.
Brunner Strasse 59
A-1235 Vienna (AT)
(84) AT

(72) Inventor: Nadelson, Jeffrey
12 Benedict Crescent
Denville, N.J., 07834 (US)

(56) References cited:
Chemical Abstracts, vol. 49, no. 6, 25 March 1955, Columbus, Ohio (USA); G.SHAW et al.: "Isoxazolinones. VI. The hydrogenation of 5-aminoisoxazoles. A new synthesis of pyrimidines", column 2, abstract 3978g

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 084 292 B1

Courier Press, Leamington Spa, England.

(56) References cited:

Chemical Abstracts, vol. 49, no. 14, 25 July 1955, Columbus, Ohio (USA); F.J.VILLANI et al.: "Amides of ethylenediamines. II. Substituted cinnamamides as local anesthetic agents", column 2, abstract 9564f

**Description**

The present invention relates to N,N-disubstituted alkenamides and phenylalkenamides, processes for their production, pharmaceutical compositions containing them and their use as pharmaceuticals in particular as anti-diabetics.

In particular the invention relates to compounds of formula I

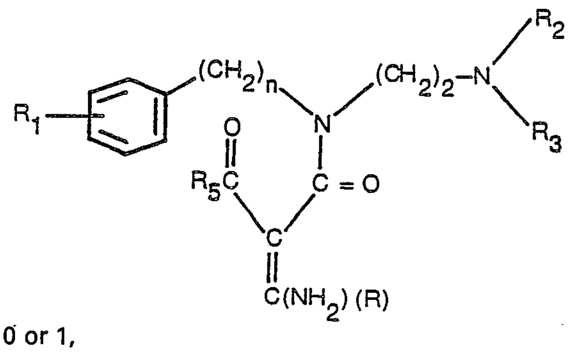

wherein n represents 0 or 1,

R represents $C_{1-4}$alkyl or the group 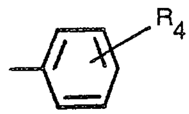 ,

$R_2$ and $R_3$ represent independently $C_{1-4}$alkyl or together with the nitrogen atom to which they are attached pyrrolidino, piperidino, hexamethyleneimino or morpholino,

$R_1$ and $R_4$ represent independently halogen, $C_{1-4}$alkyl or $C_{1-4}$alkoxy, and

$R_5$ represents hydrogen or $C_{1-6}$alkyl, and acid addition salts thereof.

Unless otherwise stated, halogen stands for fluoro or chloro.

The compounds of formula I can exist in the form of cis/trans geometric isomers about the C=C double bond. They may also exist in additional tautomeric forms to that of formula I as follows

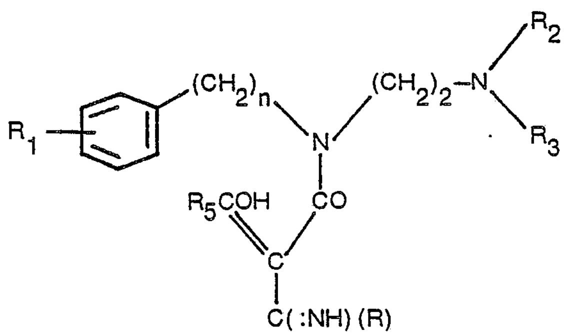

Ia

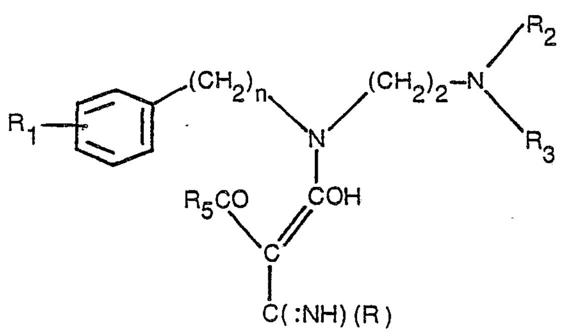

Ib

All of the tautomeric forms and the geometrical isomers thereof and their acid addition salts form part of the invention.

The compounds of formula I may be prepared according to the invention by reducing a compoujd of formula II

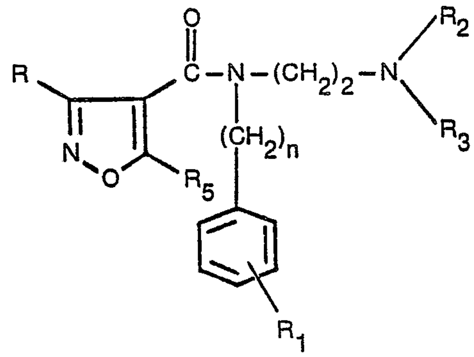

II

wherein R, $R_1$, $R_2$, $R_3$, $R_5$ and n are defined above.

Reduction may be carried out in an inert solvent such as a lower alkanol e.g. methanol or ethanol or e.g. dimethylformamide and is preferably performed under hydrogen in the presence of an hydrogenation catalyst such as palladium on carbon, platinum oxide or Raney nickel, preferably 10% palladium on carbon. Pressures of 97—690 kPa (14 psi to 100 psi), especially 242—483 kPa (35 to 70 psi) and temperatures of about 20 to 100°C, especially 20° to 70°C are usually employed.

The compounds of formula II may be prepared by reacting a compound of formula III

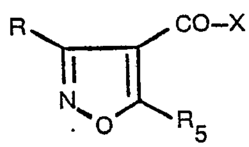

III

with a compound of formula IV

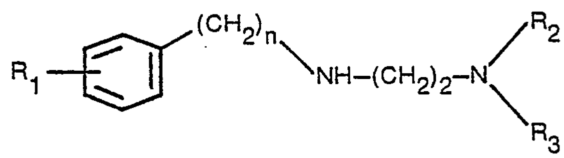

IV

whereby in the formulae III and IV R, $R_1$, $R_2$, $R_3$, $R_5$ and n are as defined above and X represents chloro or bromo.

The reaction may be carried out in the presence of an inert solvent or solvent mixture such as an aromatic hydrocarbon e.g. benzene or toluene; a chlorinated hydrocarbon e.g. methylene dichloride or chloroform or an ether e.g. diethylether or as preferred solvent tetrahydrofuran and mixtures thereof. The reaction is further preferably carried out in the presence of an acid-binding agent such as diisopropylethylamine, pyridine, potassium or sodium bicarbonate or most preferably triethylamine and at temperatures between −30° and 100°C, preferably 20° to 30°C. The compounds of formula II are new and also form part of the invention. Compounds of formulae III and IV are either known or can be prepared analogously to known methods.

Isolation of end products and if desired intermediates is carried out in conventional manner e.g. evaporation, recrystallisation.

The compounds of formula I can be recovered in free base form or in the form of their acid addition salts. Free base forms and acid addition salt forms may be prepared or interconverted in conventional manner.

The compounds of formula I possess pharmacological activity. In particular they possess hypoglycemic activity as is indicated by the lowering of blood glucose levels in male Wistar rats weighing 200 to 210 grams. The test animals are fasted in groups of 5 for 16 hours and then are dosed with 25 to 200 milligrams per kilogram of animal body weight of the compound orally. The animals are maintained on water; and two hours after the test compound is administered, the rats are anesthetized with ether and blood is collected via cardiac puncture. The blood samples are placed in an autoanalyzer potassium ferric cyanide N-2b method. These glucose levels are then compared with the glucose level of a control group, which receives orally 0.5% carboxymethyl cellulose and is run concurrently. The compounds are thus indicated for use as hypoglycemic agents in the treatment of diabetes.

The compounds of formula I are also capable of impeding or inhibiting postprandial hyperglycemia as indicated in oral starch loading tests on male Wistar rats weighing 160—180 grams as described in EP Appln. 81810131.3 (Publn. No. 0038298).

The compounds are thus also indicated for use in the treatment of diabetes by inhibiting or impeding post-prandial hyperglycemia.

As indicated suitable daily dosage for use as hypoglycemic agents in the treatment of diabetes is from about 100 to 3000 mg preferably 100 to 2000 mg suitably administered in divided dose of 25 to 1500 mg preferably 25 to 1000 mg, two to four times daily or in retard form.

As indicated suitable daily dosage for inhibiting or impeding post-prandial hyperglycemia in the treatment of diabetes is from about 100 to 3000 mg preferably 100 to 2000 mg preferably administered at mealtimes e.g. three times a day in divided dosages of from about 40 to 1000 mg preferably 40 to 700 mg, particularly before a carbohydrate-rich meal.

The invention therefore also concerns compounds of formula I which can be applied in a method of treating diabetes by lowering blood sugar levels or inhibiting or impeding post-prandial hyperglycemia by administration and also to compounds of formula I for use as pharmaceuticals e.g. as hypoglycemic agents or as agents for inhibiting or impeding post-prandial hyperglycemia.

The compounds of formula I may be administered in free base form or in the form of pharmaceutically acceptable acid addition salts, which salt forms have the same order of activity as the free forms. Examples of such salts are inorganic salts such as hydrochloride, hydrobromide, hydroiodide, phosphate (including hydrogen phosphate), metaphosphate and sulphate (including hydrogen sulphate) and organic salts such as acetate, maleate, fumarate and the like.

The compounds of formula I or their pharmaceutically acceptable acid addition salts may be administered alone, or in admixture with a pharmaceutically acceptable diluent or carrier, and, optionally other excipients, and administered orally in such forms as tablets, dispersible tablets, granules, capsules, syrups and elixirs or parenterally as solutions e.g. sterile injectable aqueous solutions.

The preferred pharmaceutical compositions from the stand-point of ease of preparation and administration are solid compositions, particularly tablets and hard-filled or liquid-filled capsules.

Such compositions also form part of the invention.

An Example of a particular compound group is that wherein in formula I $R_2$ and $R_3$ represent independently $C_{1-4}$alkyl, n represents 0 and R and $R_1$ are as defined above, and acid addition salts thereof.

The following meanings for individual substituents and or combinations thereof are preferred

R = a) $C_{1-4}$alkyl, especially methyl or ethyl,

b) 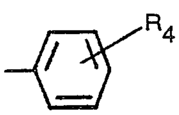

wherein $R_4$ = hydrogen or halogen (especially fluorine) in particular hydrogen,

$R_1$ = a) hydrogen,
b) halogen especially fluorine,
c) $C_{1-4}$alkyl, especially methyl,
d) $C_{1-4}$alkoxy, especially methoxy,

$R_2$, $R_3$ = a) $C_{1-4}$alkyl, especially methyl or ethyl,
b) pyrrolidino, piperidino, hexamethylenimino or morpholino, especially piperidino or morpholino,

$R_5$ = a) hydrogen,
b) straight chain alkyl,
c) $C_{1-3}$alkyl,
d) n-propyl, ethyl and especially methyl.

Particularly preferred individual compounds are:

2 - (aminophenylmethylene) - N - (2 - dimethylaminoethyl) - N - phenyl - 3 - oxo - butanamide (R = phenyl; $R_1$ = H; $R_2 = R_3 = R_5 = CH_3$; n = 0);

2 - acetyl - 3 - amino - N - (2 - dimethylaminoethyl) - N - (o - fluorophenyl) - pent - 2 - enamide (R = ethyl; $R_1$ = o-fluoro; $R_2 = R_3 = R_5 = CH_3$; n = 0 and especially

2 - acetyl - 3 - amino - N - (2 - dimethylaminoethyl) - N - phenyl - pent - 2 - enamide (R = ethyl; $R_1$ = H; $R_2 = R_3 = R_5 = CH_3$; n = 0).

The following examples, in which all temperatures are in °C and room temperature is 20—30°C, illustrate the invention.

Example

*2-(Aminophenylmethylene)-N-(2-dimethylaminoethyl)-N-phenyl-3-oxo-butanamide (Compound No. 1)*

a) N-Phenyl-N-(2-dimethylaminoethyl)-5-methyl-3-phenyl-4-isoxazole carboxamide (compound a)i))

A mixture of 24.6 g (0.15 mol) of N,N-dimethyl-N'-phenylethylenediamine and 18.2 g (0.18 mol) of triethylamine in 500 millilitres of tetrahydrofuran (THF) is cooled to 0°C and 39.8 g (0.18 mol) of 5-methyl-3-phenyl-4-isoxazole carbonyl chloride in 100 millilitres of tetrahydrofuran is added dropwise. After the addition is complete, the mixture is warmed to room temperature and stirred for 4 hours. The resulting suspension is filtered and the THF removed in vacuo. The residue is dissolved in methylene chloride and washed with 2N sodium hydroxide and water and then dried over magnesium sulfate. The mixture is

filtered and the solvent removed by evaporation. The residue is then treated with hexane to yield crystals of N-phenyl-N-(2-dimethylaminoethyl)-5-methyl-3-phenyl-4-isoxazole carboxamide; m.p. 88—89°.

The following intermediates of formula II in which $R_5$ is methyl may be obtained analogously from appropriate starting materials.

| Compound | R | $R_1$ | $R_2$ | $R_3$ | n | Physical data |
|---|---|---|---|---|---|---|
| a)ii) | ethyl | H | $CH_3$ | $CH_3$ | O | m.p. 140—142° |
| a)iii) | phenyl | p-fluoro | $CH_3$ | $CH_3$ | O | m.p. 62—64° |
| a)iv) | phenyl | p-methyl | $CH_3$ | $CH_3$ | O | oil |
| a)v) | phenyl | p-methoxy | $CH_3$ | $CH_3$ | O | Oil |
| a)vi) | phenyl | H | morpholino | | O | m.p. 115—117° |
| a)vii) | phenyl | H | piperidino | | O | m.p. 83—84° |
| a)viii) | phenyl | H | $CH_3$ | $CH_3$ | 1 | m.p. 85—87° |
| a)ix) | phenyl | H | $C_2H_5$ | $C_2H_5$ | O | oil |
| a)x) | ethyl | m-fluoro | $CH_3$ | $CH_3$ | O | m.p. 162—164°(HCl salt) |
| a)ix) | ethyl | p-fluoro | $CH_3$ | $CH_3$ | O | oil |
| a)xii) | ethyl | o-fluoro | $CH_3$ | $CH_3$ | O | oil |
| a)xiii) | ethyl | H | $CH_3$ | $CH_3$ | 1 | m.p. 69—71° (citrate) |

The intermediates of formula II in which R is ethyl, $R_1$ is hydrogen, $R_2$ and $R_3$ are methyl and $R_5$ is hydrogen, ethyl or n-propyl, are obtained by analogous methods from appropriate starting materials as oils.

b) 2-(Aminophenylmethylene)-N-(2-dimethylaminoethyl)-N-phenyl-3-oxo-butanamide (Compound No. 1)

A mixture of 35 g (0.1 mol) of N-phenyl-N-(2-dimethylaminoethyl)-5-methyl-3-phenyl-4-isoxazole carboxamide and 3.8 g of 10% palladium on carbon in 200 millilitres of dimethylformamide is hydrogenated at 50 psi and 50—60° overnight. The mixture is cooled, filtered, and the filtrate evaporated in vacuo. The residue obtained is then treated with hexane and the resulting crystals triturated with ether to give 2-(-aminophenylmethylene)-N-(2-dimethylaminoethyl)-N-phenyl-3-oxo-butanamide; m.p. 81—83°.

The following compounds may be obtained analogously from appropriate intermediates.

| Compound No. | R | $R_1$ | $R_2$ | $R_3$ | $R_5$ | n | Physical data |
|---|---|---|---|---|---|---|---|
| 2 | ethyl | H | $CH_3$ | $CH_3$ | $CH_3$ | 0 | m.p. 103—105° |
| 3 | phenyl | p-fluoro | $CH_3$ | $CH_3$ | $CH_3$ | 0 | m.p. 99—100° |
| 4 | phenyl | p-methyl | $CH_3$ | $CH_3$ | $CH_3$ | 0 | m.p. 107—108° |
| 5 | phenyl | p-methoxy | $CH_3$ | $CH_3$ | $CH_3$ | 0 | m.p. 110—111° |
| 6 | phenyl | H | morpholino | | $CH_3$ | 0 | m.p. 138—139° |
| 7 | phenyl | H | piperidino | | $CH_3$ | 0 | m.p. 64—65° |
| 8 | phenyl | H | $CH_3$ | $CH_3$ | $CH_3$ | 1 | m.p. 105—107° |
| 9 | phenyl | H | $C_2H_5$ | $C_2H_5$ | $CH_3$ | 0 | m.p. 62—63° |
| 10 | ethyl | m-fluoro | $CH_3$ | $CH_3$ | $CH_3$ | 0 | m.p. 93—94.5° |
| 11 | ethyl | p-fluoro | $CH_3$ | $CH_3$ | $CH_3$ | 0 | m.p. 89—91° |
| 12 | ethyl | o-fluoro | $CH_3$ | $CH_3$ | $CH_3$ | 0 | m.p. 98.5—100° |
| 13 | ethyl | H | $CH_3$ | $CH_3$ | $CH_3$ | 1 | m.p. 123—125° |
| 14 | ethyl | H | $CH_3$ | $CH_3$ | H | 0 | m.p. 163—165.5° |
| 15 | ethyl | H | $CH_3$ | $CH_3$ | $C_2H_5$ | 0 | m.p. 140—141° |
| 16 | ethyl | H | $CH_3$ | $CH_3$ | $C_3H_7$ | 0 | m.p. 164—165° |

**Claims for the Contracting States: BE CH DE FR GB IT LI LU NL SE**

1. Compounds of formula I

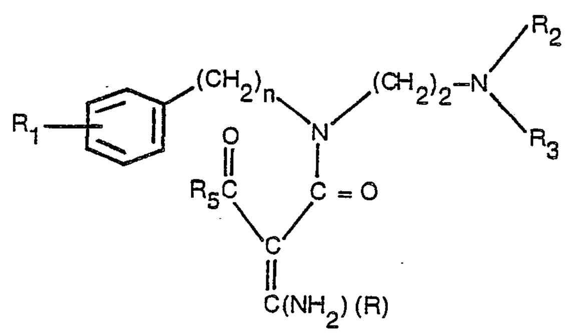

wherein n represents 0 or 1

R represents $C_{1-4}$alkyl or the group 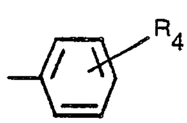 ,

wherein $R_2$ and $R_3$ represent independently $C_{1-4}$alkyl or toether with the nitrogen atom to which they are attached pyrrolidino, piperidino, hexamethyleneimino or morpholino

$R_1$ and $R_4$ represent independently halogen, $C_{1-4}$alkyl or $C_{1-4}$alkoxy and

$R_5$ represents hydrogen or $C_{1-6}$alkyl, and acid addition salts thereof.

2. A compound as claimed in Claim 1 wherein $R_2$ and $R_3$ represent independently $C_{1-4}$alkyl and acid addition salts thereof.

3. A compound as claimed in Claim 1 or 2 wherein n represents 0 and acid addition salts thereof.

4. A compound as claimed in any one of Claims 1 to 4 wherein $R_5$ represents methyl.

5. A compound selected from 2 - (aminophenylmethylene) - N - (2 - diethylaminoethyl) - N - phenyl - 3 -

oxo - butanamide; 2 - acetyl - 3 - amino - N - (2 - dimethylaminoethyl) - N - (m - fluorophenyl) - pent - 2 - enamide; 2 - (aminophenylmethylene) - N - (2 - piperidinoethyl) - N - phenyl - 3 - oxo - butanamide; 2 - (aminophenylmethylene) - N - (2 - dimethylaminoethyl) - N - (p - fluorophenyl) - 3 - oxo - butanamide; 2 - (aminophenylmethylene) - N - (2 - dimethylaminoethyl) - N - phenyl - 3 - oxo - butanamide; 2 - acetyl - 3 - amino - N - (2 - dimethylaminoethyl) - N - (o - fluorophenyl) - pent - 2 - enamide and 2 - acetyl - 3 - amino - N - (2 - dimethylaminoethyl) - N - phenyl - pent - 2 - enamide, and acid addition salts of each of these.

6. A pharmaceutical composition comprising a compound as claimed in any one of Claims 1 to 5 in free base form or in the form of a pharmaceutically acceptable acid addition salt thereof in admixture with a pharmaceutically acceptable diluent or carrier.

7. A compound as claimed in any one of Claims 1 to 5 in free base form or in the form of a pharmaceutically acceptable acid addition salt for use as a pharmaceutical in particular as a hypoglycemic agent and/or an agent for inhibiting or impeding post-prandial hyperglycemia.

8. A process for the production of compounds of formula I which comprises reducing a compound of formula II

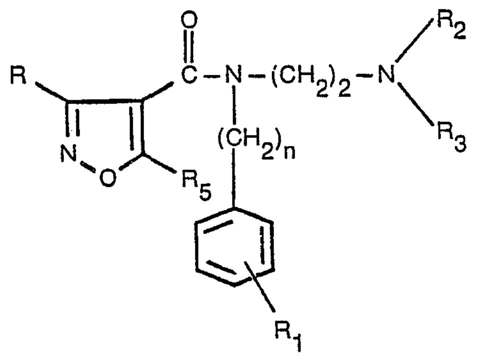

II

wherein R, $R_1$, $R_2$, $R_3$, $R_5$ and n are as defined in Claim 1, and recovering the compound thus obtained in free base or acid addition salt form.

9. A compound of formula II

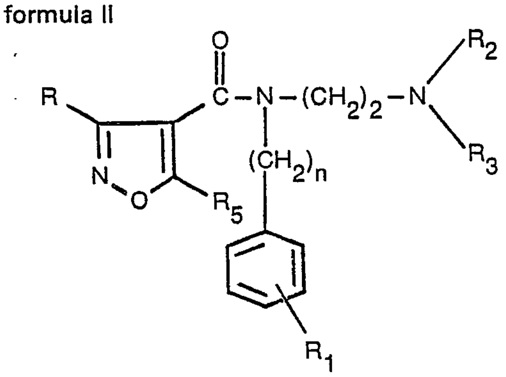

II

wherein R, $R_1$, $R_2$, $R_3$, $R_5$ and n are as defined in Claim 1.

**Claims for the Contracting State: AT**

1. Process for preparing a compound of formula I

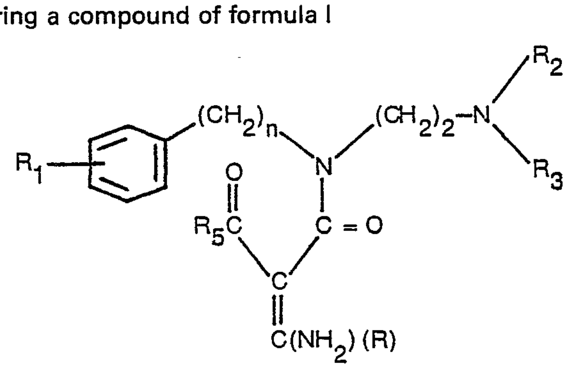

wherein n represents 0 or 1,

R represents $C_{1-4}$alkyl or the group 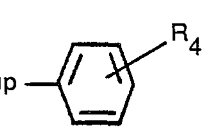 ,

$R_2$ and $R_3$ represent independently $C_{1-4}$alkyl or together with the nitrogen atom to which they are attached pyrrolidino, piperidino, hexamethyleneimino or morpholino,

$R_1$ and $R_4$ represent independently halogen, $C_{1-4}$alkyl or $C_{1-4}$alkoxy, and

$R_5$ represents hydrogen or $C_{1-6}$alkyl, and acid addition salts thereof, which comprises reducing a compound of formula II

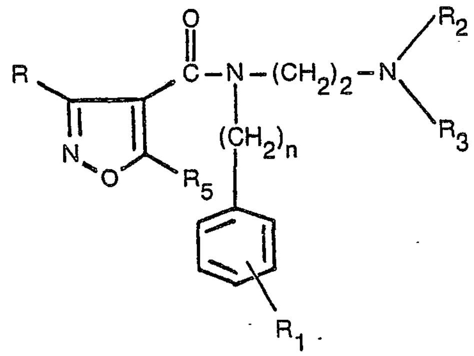

II

wherein R. $R_1$, $R_2$, $R_3$, $R_5$ and n are as defined in Claim 1, and recovering the compound thus obtained in free base or acid addition salt form.

2. A process as claimed in Claim 1 wherein $R_2$ and $R_3$ represent independently $C_{1-4}$alkyl.

3. A process as claimed in Claim 1 or 2 wherein n represents 0.

4. A process as claimed in any one of Claims 1 to 4 wherein $R_5$ represents methyl.

5. A process according to Claim 1 wherein the compound prepared is selected from 2 - (aminophenylmethylene) - N - (2 - diethylaminoethyl) - N - phenyl - 3 - oxo - butanamide; 2 - acetyl - 3 - amino - N - (2 - dimethylaminoethyl) - N - (m - fluorophenyl) - pent - 2 - enamide; 2 - (aminophenylmethylene) - N - (2 - piperidinoethyl) - N - phenyl - 3 - oxo - butanamide; 2 - (aminophenylmethylene) - N - (2 - dimethylaminoethyl) - N - (p - fluorophenyl) - 3 - oxo - butanamide; 2 - (aminophenylmethylene) - N - (2 - di - methylaminoethyl) - N - phenyl - 3 - oxo - butanamide; 2 - acetyl - 3 - amino - N - (2 - dimethylaminoethyl) - N - (o - fluorophenyl) - pent - 2 - enamide and 2 - acetyl - 3 - amino - N - (2 - dimethylaminoethyl) - N - phenyl - pent - 2 - enamide, and acid addition salts of each of these.

6. A pharmaceutical composition comprising a compound prepared according to any one of Claims 1 to 5 in free base form or in the form of a pharmaceutically acceptable acid addition salt thereof in admixture with a pharmaceutically acceptable diluent or carrier.

7. A compound as prepared according to any one of Claims 1 to 5 in free base form or in the form of a pharmaceutically acceptable acid addition salt for use as a pharmaceutical in particular as a hypoglycemic agent and/or an agent for inhibiting or impeding post-prandial hyperglycemia.

8. A process for the preparation of a pharmaceutical composition comprising admixing a compound of formula I

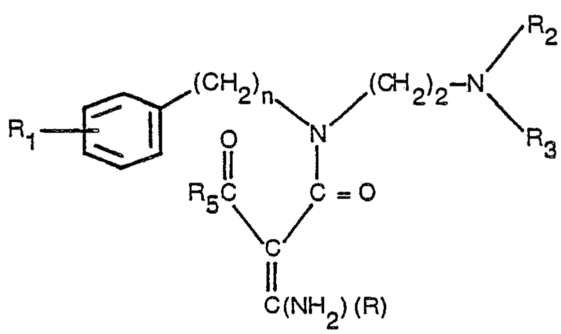

wherein n represents 0 or 1,

R represents $C_{1-4}$alkyl or the group 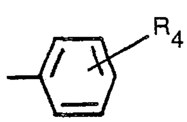 ,

$R_2$ and $R_3$ represent independently $C_{1-4}$alkyl or together with the nitrogen atom to which they are attached pyrrolidino, piperidino, hexamethyleneimino or morpholino,

$R_1$ and $R_4$ represent independently halogen, $C_{1-4}$alkyl or $C_{1-4}$alkoxy, and

$R_5$ represents hydrogen or $C_{1-6}$alkyl, in free base form or in the form of a pharmaceutically acceptable acid addition salt thereof in admixture with a pharmaceutically acceptable diluent or carrier.

**Patentansprüche für die Vertragstaaten: BE CH DE FR GB IT LI LU NL SE**

1. Verbindungen der Formel I

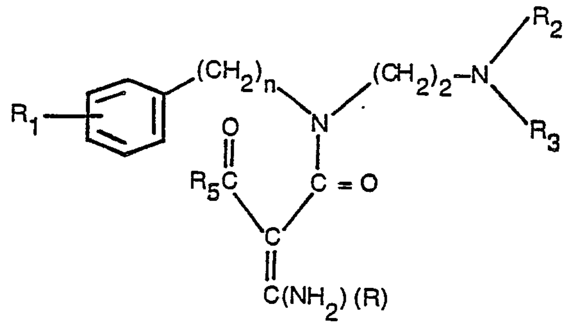

I

worin n 0 oder 1 darstellt

R $C_{1-4}$-Alkyl oder die Gruppe 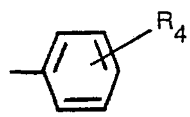 ,

darstellt,

worin $R_2$ und $R_3$ unabhängig $C_{1-4}$Alkyl oder zusammen mit dem Stickstoffatom, an das sie gebunden sind, Pyrrolidino, Piperidino, Hexamethylenimino oder Morpholino darstellen,

$R_1$ und $R_4$ unabhängig Halogen, $C_{1-4}$-Alkyl oder $C_{1-4}$Alkoxy darstellen und $R_5$ Wasserstoff oder $C_{1-6}$Alkyl darstellt, und Säureadditionssalze davon.

2. Eine Verbindung wie im Anspruch 1 beansprucht, worin $R_2$ und $R_3$ unäbhängig $C_{1-4}$-Alkyl darstellen, und Säureadditionssalze davon.

3. Eine Verbindung wie im Anspruch 1 oder 2 beansprucht, worin n 0 darstellt, und Säureadditionssalze davon.

4. Eine Verbindung wie in einem der Ansprüche 1 bis 4 beansprucht, worin $R_5$ methyl darstellt.

5. Eine Verbindung ausgewählt aus 2-(Aminophenylmethylen) - N - (2 - diethylaminoethyl) - N - phenyl - 3 - oxo - butanamid; 2 - Acetyl - 3 - amino - N - (2 - diethylaminoethyl) - N - (m - fluorophenyl) - pent - 2 - enamid; 2 - (Aminophenylmethylene) - N - (2 - piperidinoethyl) - N - phenyl - 3 - oxo - butanamid; 2 - (Aminophenylmethylen) - N - (2 - dimethylaminoethyl) - N - (p - fluorophenyl) - 3 - oxo - butanamid; 2 - (Aminophenylmethylen) - N - (2 - di - methylaminoethyl) - N - phenyl - 3 - oxo - butanamid; 2 - Acetyl - 3 - amino - N - (2 - dimethylaminoethyl) - N - (o - fluorophenyl) - pent - 2 - enamid und 2 - Acetyl - 3 - amino - N - (2 - dimethylaminoethyl) - N - phenyl - pent - 2 - enamide, sowie die Säureadditionssalze von jeder dieser Verbindungen.

6. Eine pharmazeutische Zusammensetzung enthaltend eine Verbindung wie in einen der Ansprüche 1 bis 5 beansprucht in Form der freien Base oder in Form eines pharmazeutisch annehmbaren Säureadditionssalzes, davon unter Beimischung eines pharmazeutisch annehmbaren Verdünners oder Trägers.

7. Eine Verbindung wie in einem der Ansprüche 1 bis 5 beansprucht in Form der freien Base oder in Form eines pharmazeutisch annehmbaren Säureadditionssalzes zur Verwendung als ein Pharmazeutikum insbesondere als ein hypoglykämisches Mittel und/oder ein Mittel zur Hemmung oder zum Verhindern von Hyperglykämie nach dem Essen.

8. Verfahren zur Herstellung von Verbindungen der Formel I bestehend aus Reduktion einer Verbindung der Formel II

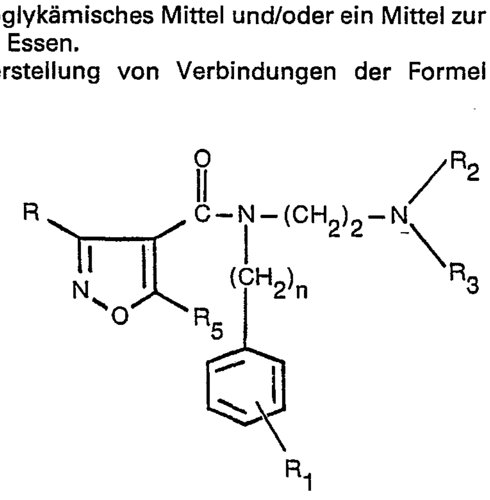

II

worin R, $R_1$, $R_2$, $R_3$, $R_5$ und n wie im Anspruch 1 definiert sind, und Wiedergewinnung der so erhaltenen Verbindung in Form der freien Base oder in Form eines Säureadditionssalzes.

9. Eine Verbindung der Formel II

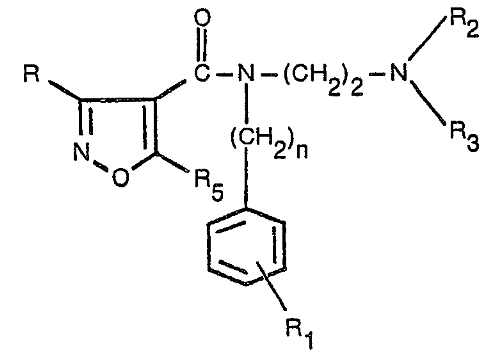

II

worin R. $R_1$, $R_2$, $R_3$, $R_5$ und n wie im Anspruch 1 definiert sind.

**Patentansprüche für den Vertragsstaat: AT**

1. Verfahren zur Herstellung von Verbindungen der Formel I

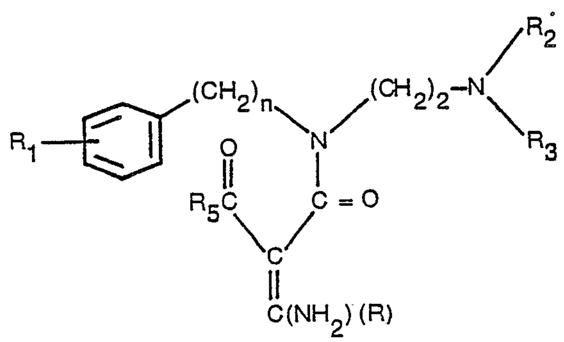

I

worin n 0 oder 1 darstellt

R $C_{1-4}$-Alkyl oder die Gruppe 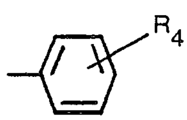 ,

darstellt,
worin $R_2$ und $R_3$ unabhängig $C_{1-4}$Alkyl oder zusammen mit dem Stickstoffatom, an das sie gebunden sind, Pyrrolidino, Piperidino, Hexamethylenimino oder Morpholino darstellen,

$R_1$ und $R_4$ unabhängig Halogen, $C_{1-4}$-Alkyl oder $C_{1-4}$Alkoxy darstellen und $R_5$ Wasserstoff oder $C_{1-6}$Alkyl darstellt, und Säureadditionssalze davon, bestehend aus Reduktion einer Verbindung der formula II

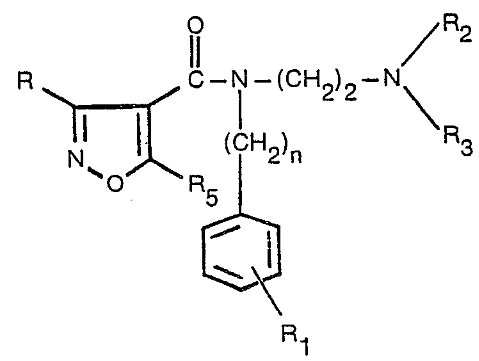

II

worin R, $R_1$, $R_2$, $R_3$, $R_5$ und n wie im Anspruch 1 definiert sind, und Wiedergewinnung der so erhalten Verbindung in Form der freien Base oder in Form eines Säureadditionssalzes.

2. Verfahren wie im Anspruch 1 beansprucht, worin $R_2$ und $R_3$ unabhängig $C_{1-4}$Alkyl darstellen.

3. Verfahren wie im Anspruch 1 oder 2 beansprucht, worin n 0 darstellt.

4. Verfahren wie in einem der Ansprüche 1 bis 4 beansprucht, worin $R_5$ Methyl darstellt.

5. Verfahren gemäss Anspruch 1, worin die hergestellte Verbindung ausgewählt wird aus 2 - (Aminophenylmethylen) - N - (2 - diethylaminoethyl) - N - phenyl - 3 - oxo - butanamid; 2 - Acetyl - 3 - amino - N - (2 - dimethylaminoethyl) - N - (m - fluorophenyl) - pent - 2 - enamid; 2 - (Aminophenylmethylene) - N -

(2 - piperidinoethyl) - N - phenyl - 3 - oxo - butanamid; 2 - (Aminophenylmethylen) - N - (2 - di - methylaminoethyl) - N - phenyl - 3 - oxo - butanamid; 2 - Acetyl - 3 - amino - N - (2 - dimethylaminoethyl) - N - (o - fluorophenyl) - pent - 2 - enamid und 2 - Acetyl - 3 - amino - N - (2 - dimethylaminoethyl) - N - phenyl - pent - 2 - enamide, sowie die Säureadditionssalze von jeder dieser Verbindungen.
2 - enamide, sowie die Säureadditionssalze von jeder dieser Verbindungen.

6. Eine pharmazeutische Zusammensetzung enthaltend eine gemäss einem der Ansprüche 1 bis 5 hergestellte Verbindung in Form der freien Base oder in Form eines pharmazeutisch annehmbaren Säureadditionssalzes davon, unter Beimischung eines pharmazeutisch annehmbaren Verdünners oder Trägers.

7. Eine gemäss einem der Ansprüche 1 bis 5 hergestellte Verbindung zur Verwendung als ein Pharmazeutikum insbesondere als ein hypoglykämisches Mittel und/oder ein Mittel zur Hemmung oder zum Verhindern von Hyperglykämie nach dem Essen.

8. Verfahren zur Herstellung einer pharmazeutischen Zusammensetzung bestehend aus Beimischung einer Verbindung der Formel I

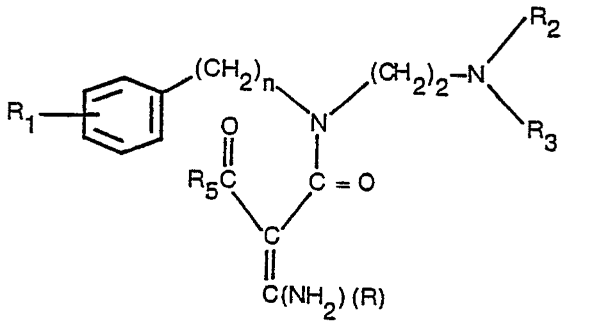

I

worin n 0 oder 1 darstellt

R $C_{1-4}$-Alkyl oder die Gruppe 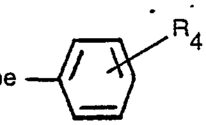 ,

darstellt,
worin $R_2$ und $R_3$ unabhängig $C_{1-4}$Alkyl oder zusammen mit dem Stickstoffatom, an das sie gebunden sind, Pyrrolidino, Piperidino, Hexamethylenimino oder Morpholino darstellen,
$R_1$ und $R_4$ unabhängig Halogen, $C_{1-4}$-Alkyl oder $C_{1-4}$Alkoxy darstellen und $R_5$ Wasserstoff oder $C_{1-6}$Alkyl darstellt, in Form der freien Base oder in Form eines pharmazeutisch annehmbaren Säureadditionssalzes davon, unter Beimischung eines pharmazeutisch annehmbaren Verdünners oder Trägers.

**Revendications pour les Etats contractants: BE CH DE FR GB IT LI LU NL SE**

1. Composés de formule I

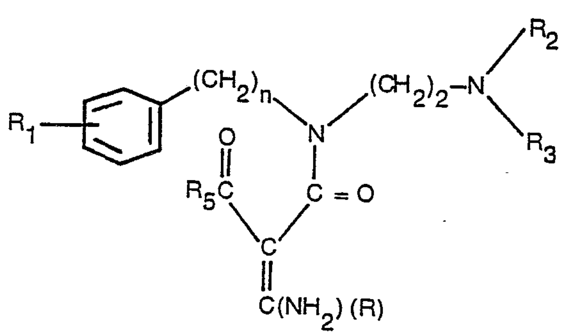

I

dans laquelle n représente 0 ou 1.

R représent alkyle en $C_1$—$C_4$ ou le groupe 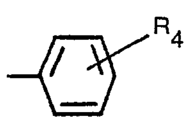 ,

dans laquelle $R_2$ et $R_3$ représentent indépendamment alkyle en $C_1$—$C_4$ ou ensemble avec l'atome d'azote auquel ils sont liés pyrrolidino, pipéridino, hexaméthylèneimino ou morpholino,
$r_1$ et $R_4$ représentent indépendamment un halogène, alkyle en $C_1$—$C_4$ ou alcoxy en $C_1$—$C_4$ et
$R_5$ représente l'hydrogène ou alkyle en $C_1$—$C_6$, et les sels d'addition d'acides de ces composés.

2. Un composé comme revendiqué à la revendication 1, dans lequel $R_{21}$ et $R_3$ représentent indépendamment alkyle en $C_1$—$C_4$ et les sels d'addition d'acides de ce composé.

3. Un composé comme revendiqué à la revendication 1 ou 2, dans lequel n représente 0 et les sels d'addition d'acides de ce composé.

4. Un composé comme revendicqué ù l'une quelconque des revendications 1 à 4, dans lequel $R_5$ représente méthyle.

5. Un composé choisi parmi le 2-(aminophénylméthylène) - N - (2 - diéthylaminoéthyl) - N - phényl - 3 - oxo - butanamide, le 2 - acétyl - 3 - amino - N - (2 - diméthylaminoéthyl) - N - (m - fluorophényl) - pent - 2 - énamide, le 2 - (aminophénylméthylène) - N - (2 - pipéridinoéthyl) - N - phényl - 3 - oxo - butanamide, le 2 - (aminophénylméthylène) - N - (2 - diméthylaminoéthyl) - N - (p - fluorophényl) - 3 - oxo - butanamide, le 2 - (aminophénylméthylène) - N - (2 - diméthylaminoéthyl) - N - phényl - 3 - oxo - butanamide, le 2 - acétyl - 3 - amino - N - (2 - diméthylaminoéthyl) - N - (o - fluorophényl) - pent - 2 - énamide et le 2 - acétyl - 3 - amino - N - (2 - diméthylaminoéthyl) - N - phényl - pent - 2 - énamide, et les sels d'addition d'acides de chacun de ces composés.

6. Une composition pharmaceutique comprenant un composé comme revendiqué à l'une quelconque des revendications 1 à 5, sous forme de base libre ou sous forme d'un sel d'addition d'acide pharmaceutiquement acceptable de ce composé, en association avec un diluant ou véhicule pharmaceutiquement acceptable.

7. Un composé comme revendiqué à l'une quelconque des revendications 1 à 5 sous forme de base libre ou sous forme d'un sel d'addition d'acide pharmaceutiquement acceptable pour l'utilisation comme médicament en particulier comme agent hypoglycémiant et/ou comme agent pour inhiber ou empêcher l'hyperglycémie post-prandiale.

8. Un procédé de préparation des composés de formule I qui comprend la réduction d'un composé de formule II

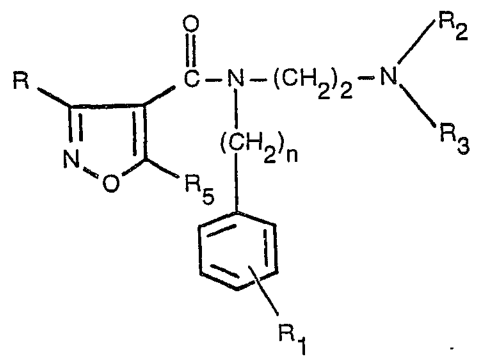

II

dans laquelle R, $R_1$, $R_2$, $R_3$, $R_5$ et n sont tels que définis à la revendication 1, et la récupération du composé ainsi obtenu sous forme de base libre ou sous forme d'un sel d'addition d'acide.

9. Un composé de formule II

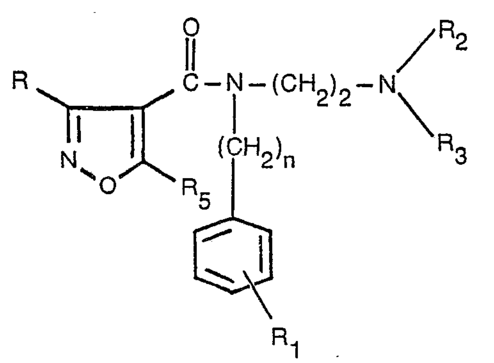

II

dans laquelle R, $R_1$, $R_2$, $R_3$, $R_5$ et n sont tels que définis à la revendication 1.

**Revendications Pour l'Etat contractant: AT**

1. Un procédé de préparation d'un composé de formule I

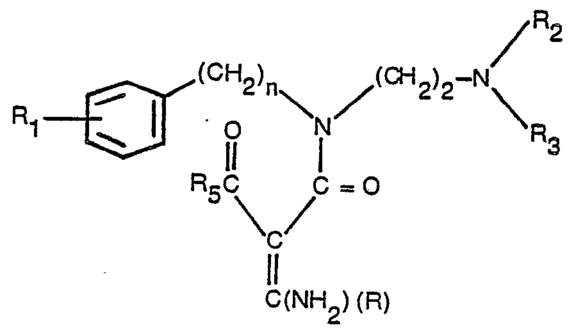

I

dans laquelle n représente 0 ou 1.

R représent alkyle en $C_1$—$C_4$ ou le groupe 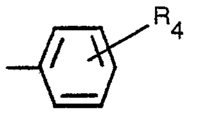 ,

$R_2$ et $R_3$ représentent indépendamment alkyle en $C_1$—$C_4$ ou ensemble avec l'atome d'azote auquel ils sont liés pyrrolidino, pipéridino, hexaméthylèneimino ou morpholino,

$R_1$ et $R_4$ représentent indépendamment un halogène, alkyle en $C_1$—$C_4$ ou alcoxy en $C_1$—$C_4$ et

$R_5$ représente l'hydrogène ou alkyle en $C_1$—$C_6$, et des sels d'addition d'acides de ce composé, qui comprend la réduction d'un composé de formule II

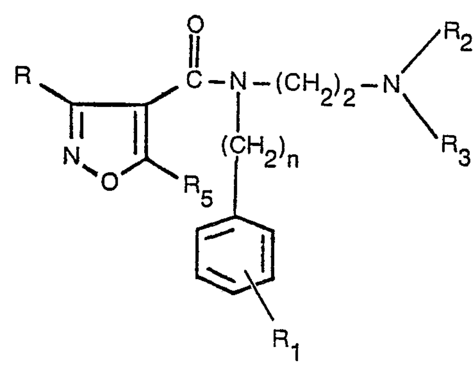

II

dans laquelle R. $R_1$, $R_2$, $R_3$ $R_5$ et n sont tels que définis à la revendication 1, et la récupération du composé ainsi obtenu sous forme de base libre ou sous forme d'un sel d'addition d'acide.

2. Un procédé comme revendiqué à la revendication 1, dans lequel $R_2$ et $R_3$ représentent indépendamment alkyle en $C_1$—$C_4$.

3. Un procédé comme revendiqué à la revendication 1 ou 2, dans lequel n représente 0.

4. Un procédé comme revendiqué à l'une quelconque des revendications 1 à 4, dans lequel $R_5$ représente méthyle.

5. Un procédé selon la revendication 1, dans lequel le composé préparé choisi parmi le 2 - (aminophénylméthylène) - N - (2 - diéthylaminoéthyl) - N - phényl - 3 - oxo - butanamide, le 2 - acétyl - 3 - amino - N - (2 - diméthylaminoéthyl) - N - (m - fluorophényl) - pent - 2 - énamide, le 2 - (aminophénylméthylène) - N - (2 - pipéridinoéthyl) - N - phényl - 3 - oxo - butanamide, le 2 - (aminophénylméthylène) - N - (2 - diméthylaminoéthyl) - N (p - fluorophényl) - 3 oxo - butanamide, le 2 - (aminophénylméthylène) - N - (2 - diméthylaminoéthyl) - N - phényl - 3 - oxo - butanamide, le 2 - acétyl - 3 - amino - N - (2 - diméthylaminoéthyl) - N - (o - fluorophényl) - pent - 2 - énamide et le 1 - acétyl - 3 - amino - N - (2 - diméthylaminoéthyl) - N - phényl - pent - 2 - énamide, et les sels d'addition d'acides de chacun de ces composés.

6. Une composition pharmaceutiquement comprenant un composé préparé selon l'une quelconqe des revendications 1 à 5, sous forme de base libre ou sous forme d'un sel d'addition d'acide pharmaceutiquement acceptable de ce composé, en association avec un diluant ou véhicule pharmaceutiquement acceptable.

7. Un composé préparé selon l'une quelconque des revendications 1 à 5 sous forme de base libre ou sous forme d'un sel d'addition d'acide pharmaceutiquement acceptable pour l'utilisation comme

médicament, en particulier comme agent hypoglycémiant et/ou comme agent pour inhiber ou empêcher l'hyperglycémie post-prandiale.

8. Un procédé de préparation d'une composition pharmaceutiquement comprenant l'admixtion d'un composé de formule I

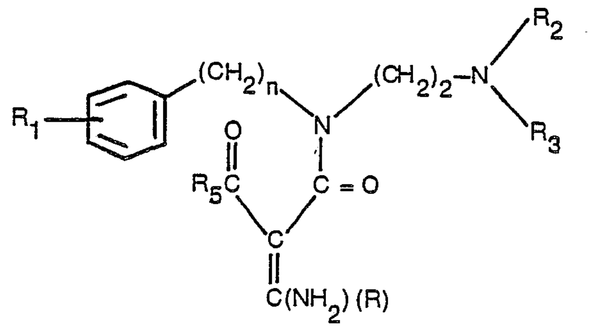

I

dans laquelle n représente 0 ou 1.

R représente alkyle en $C_1$—$C_4$ ou le groupe 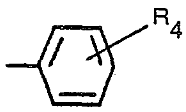 ,

$R_2$ et $R_3$ représentent indépendamment alkyle en $C_1$—$C_4$ ou ensemble avec l'atome d'azote auquel ils sont liés pyrrolidino, pipéridino, hexaméthylèneimino ou morpholino,

$R_1$ et $R_4$ représentent indépendamment un halogène, alkyle en $C_1$—$C_4$ ou alcoxy en $C_1$—$C_4$ et

$R_5$ représente l'hydrogène ou alkyle en $C_1$—$C_6$, sous forme de base libre ou sous forme d'un sel d'addition d'acide pharmaceutiquement acceptable de ce composé en mélange avec un diluant ou véhicule pharmaceutiquement acceptable.